(19) Europäisches Patentamt
European Patent Office
Office européen des brevets

(11) Veröffentlichungsnummer: **0 047 923**
**B1**

(12) # EUROPÄISCHE PATENTSCHRIFT

(45) Veröffentlichungstag der Patentschrift: **09.05.84**

(21) Anmeldenummer: **81106884.0**

(22) Anmeldetag: **03.09.81**

(51) Int. Cl.³: **C 07 D 217/22**, C 07 D 401/04, C 07 D 401/12, C 07 D 405/02, C 07 D 409/12, C 07 D 413/12, A 61 K 31/47, A 61 K 31/495, A 61 K 31/505, A 61 K 31/55

(54) **Isochinolinderivate, Verfahren zu ihrer Herstellung und sie enthaltende pharmazeutische Zubereitungen.**

(30) Priorität: **10.09.80 DE 3034001**

(43) Veröffentlichungstag der Anmeldung: **24.03.82 Patentblatt 82/12**

(45) Bekanntmachung des Hinweises auf die Patenterteilung: **09.05.84 Patentblatt 84/19**

(84) Benannte Vertragsstaaten: **AT BE CH DE FR GB IT LI LU NL SE**

(56) Entgegenhaltungen:
**DE - A - 2 030 675**
**DE - A - 2 818 403**
**DE - B - 2 420 012**
**DE - B - 2 503 961**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Konz, Elmar, Dr., Brüningstrasse 9, D-6233 Kelkheim Taunus (DE)**
Erfinder: **Hock, Franz, Dr., Altstadt 19, D-6110 Dieburg (DE)**
Erfinder: **Kaiser, Joachim, Dr., Egerländer Strasse 8, D-6233 Kelkheim Taunus (DE)**
Erfinder: **Kruse, Hansjörg, Dr., Feldbergstrasse 70, D-6233 Kelkheim Taunus (DE)**

**EP 0 047 923 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

3-Dialkylaminoalkylaminoisochinoline sind in der Literatur beschrieben (J. Med. chem. 13, (1970), 999–1002), doch wurden keine biologischen Wirkungen angegeben. 3,4-Dihydroisochinoline mit basischen Seitenketten in 1-Stellung des Isochinolinrings weisen antiarrhythmische Eigenschaften auf (BE 764 133 und DE 2 102 794).

Es wurden in 3-Stellung basisch substituierte Isochinoline gefunden, die wertvolle pharmakologische Wirkungen auf das zentrale Nervensystem und das Herz-Kreislauf-System ausüben.

Gegenstand der Erfindung sind Isochinolinderivate der allgemeinen Formel I

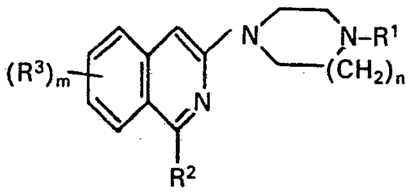

(I)

worin bedeuten:

m eins oder zwei, n eins oder zwei,

$R^1$ a) Wasserstoff,

b) $C_1$–$C_6$-Alkyl, geradkettig oder verzeigt, gegebenenfalls substituiert mit Hydroxy, $C_1$–$C_4$-Alkoxy- oder $C_3$–$C_6$-Cycloalkyl,

c) Thienyl, Furyl, Pyridyl, Pyrimidyl,

d) Formyl oder $C_1$–$C_6$-Alkoxycarbonyl,

e) Phenyl, das einfach, zweifach oder dreifach substituiert sein kann mit $C_1$–$C_6$-Alkyl, $C_1$–$C_6$-Alkoxy, Methylendioxy, Hydroxy, Nitro, Amino, Trifluormethyl oder Halogen,

f) $-(CH_2)_q-COR^4$, worin q 0, 1, 2, 3 oder 4 und $R^4$ Thienyl, Furyl, Pyridyl oder ein wie unter e) angegebener, gegebenenfalls substituierter Phenylrest ist,

g) 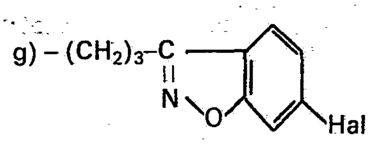 ,

wobei Hal Halogen bedeutet;

$R^2$ Wasserstoff oder $C_1$–$C_6$-Alkyl,

$R_3$ Wasserstoff, Halogen, Hydroxy-, $C_1$–$C_6$-Alkyl, $C_1$–$C_6$-Alkoxy, Benzyloxy, Methylendioxy, Äthylendioxy, Nitro oder Amino, sowie deren Salze mit physiologisch verträglichen Säuren.

Als physiologisch verträgliche Säuren kommen in Betracht anorganische Säuren wie Salzsäure, Schwefelsäure, Phosphorsäure, Bromwasserstoffsäure, oder organische Säuren, wie Ameisensäure, Essigsäure, Milchsäure, Zitronensäure, Weinsäure, Oxalsäure, Maleinsäure, Fumarsäure, Methansulfonsäure, 1,8-Naphthalindisulfonsäure, Glutarsäure oder Glucuronsäure.

Weitere Gegenstände der Erfindung sind Verfahren zur Herstellung der Verbindungen, pharmazeutische Zubereitungen und die Verwendung der Verbindungen.

Bevorzugt sind solche Verbindungen der Formel I, worin $R^1$ Wasserstoff, $C_1$–$C_6$-Alkyl, einen Phenylrest, der einfach oder zweifach substituiert sein kann mit $C_1$–$C_4$-Alkyl, $C_1$–$C_4$-Alkoxy, Methylendioxy, Hydroxy, Trifluormethyl oder Halogen, insbesondere Fluor, Chlor oder Brom, den Rest $-(CH_2)_q-COR^4$, worin q gleich 0 oder 3 und $R^4$ einen Furyl- oder einen gegebenenfalls wie oben unter $R^1$ e) angegebenen substituierten Phenylrest oder den Rest

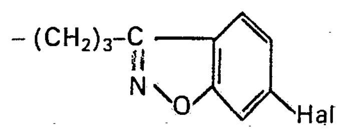 ,

wobei Hal für Halogen, insbesondere für Fluor, Chlor oder Brom steht, bedeuten, und worin $R^2$ Wasserstoff oder $C_1$–$C_4$-Alkyl, und $R^3$ Wasserstoff, Halogen, insbesondere Fluor, Chlor oder Brom, Hydroxy, $C_1$–$C_4$-Alkyl oder $C_1$–$C_4$-Alkoxy in 6- und/oder 7-Stellung bedeuten. In diesen Definitionen der Substituenten der bevorzugten Verbindungen bedeuten $C_1$–$C_4$-Alkyl jeweils Methyl, Äthyl, n-Propyl, i-Propyl, n-Butyl, i-Butyl und tert.-Butyl und $C_1$–$C_4$-Alkoxy die entsprechenden sauerstoffhaltigen Reste.

Das Verfahren zur Herstellung der Isochinolinderivate der Formel I ist dadurch gekennzeichnet, dass man a) eine Verbindung der Formel II

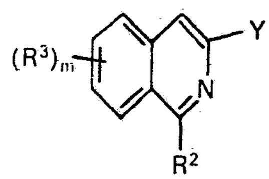

(II)

worin Y Halogen, vorzugsweise Chlor oder Brom, oder Alkyloxy oder Alkylthio mit jeweils 1–4 C-Atomen im Alkylrest, vorzugsweise Methyloxy oder Methylthio, ist und $R^2$, $R^3$ und m die zur Formel I genannten Bedeutungen haben, mit einem Amin der Formel III

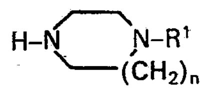

(III)

worin $R^1$ und n die zur Formel I genannten Bedeutungen haben, umsetzt,

b) eine Verbindung der Formel IV

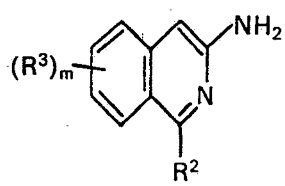

(IV)

worin $R^2$, $R^3$ und m die zur Formel I genannten Bedeutungen haben, mit einer Verbindung der Formel V

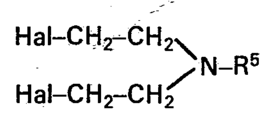

(V)

worin Hal Halogen, vorzugsweise Chlor oder Brom, und $R^5$ Wasserstoff, $C_1$–$C_6$-Alkyl oder einen wie in Formel I unter $R^1$c) oder $R^1$e) angegebenen Rest bedeutet, oder

c) eine Verbindung der Formel Ia

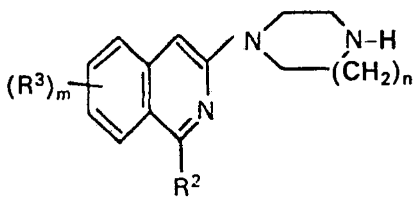

(Ia)

worin $R^2$, $R^3$, m und n die zur Formel I genannten Bedeutungen haben, mit einem Alkylierungsmittel der Formel Z–$R^6$, worin Z Jod, Chlor oder Brom und $R^6$ einen wie in Formel I unter $R^1$b angegebenen Alkylrest bedeutet, oder mit einem Chlorameisensäureester der Formel $Cl–CO_2–(C_1–C_6)$-Alkyl oder mit einer Verbindung der Formel $Cl–(CH_2)_q–COR^4$, worin $R^4$ und q die zur Formel I unter $R^1$f) genannten Bedeutungen haben, umsetzt.

Bei der Verfahrensweise a) ist es zweckmässig, mindestens zwei Äquivalente Amin zuzugeben, da ein Äquivalent Amin zur Bindung des abgespaltenen Halogenwasserstoffs gebraucht wird. Vorteilhaft kann man zur Reaktionsbeschleunigung einen bis zu 15-fachen Überschuss des Amins verwenden. Arbeitet man andererseits mit äquimolaren Mengen Amin, so wird zweckmässig ein tertiäres Amin wie Pyridin, Picolin, Lutidin, 1,4-Diazabicyclo-[5.4.0]-undec-5-en oder anorganische Basen wie Natriumcarbonat, Kaliumcarbonat oder Calciumcarbonat als Säureakzeptor hinzugegeben.

Bei Verwendung flüssiger Amine als Säureakzeptor kann auf den Zusatz von Lösungsmitteln verzichtet werden. Als Lösungsmittel kommen, sofern sie zur Umsetzung verwendet werden, indifferente, wasserfreie, organische Lösungsmittel wie Äthylenglykolmonoäthyläther, Octanol, Diäthylenglykoldimethyläther, Diäthylenglykoldibutyläther, Toluol, Xylol, Chlorbenzol, Dichlorbenzol, Trichlorbenzol, Dimethylformamid, Dimethylsulfoxid der Hexamethylphosphorsäuretriamid in Frage.

Die Reaktion wird im allgemeinen bei einer Temperatur zwischen 80° und 220°, vorzugsweise zwischen 120–180°, ausgeführt.

Die Ausgangsverbindungen der Formel II können nach bekannten Methoden durch Reduktion von Isochinolinderivaten analog Formel II, die in 1- und 3-Stellung durch Halogen substituiert sind, mit Phosphor und Jodwasserstoffsäure in Eisessig oder gegebenenfalls durch Alkylierung mit einer entsprechenden metallorganischen Verbindung (vgl. z.B. S.Gabriel, Ber. 19, 2354, Chem. pharm. Bl. (Japan) 15 (1967), 704) hergestellt werden.

Nach dem Verfahren b) werden Aminoverbindungen der Formel IV mit N-(Bis-Halogenäthyl)-aminen der Formel V kondensiert. Die Umsetzung wird zweckmässig in einem polaren Lösungsmittel wie in einem Alkohol mit 4 bis 8 C-Atomen, z.B. Isoamylalkohol, in einem Äther wie Diäthylenglykoldimethyläther oder in einem aprotischen Lösungsmittel wie Acetonitril oder Dimethylformamid bei einer Temperatur zwischen 60° und 200°C, vorzugsweise 80 und 140°C, in Gegenwart eines Säureakzeptors ausgeführt.

Die Ausgangsverbindungen IV und V für das Verfahren b) werden nach bekannten Methoden (z.B. J.org.Chem. 27, 3953, (1962) und J.Pharm. Soc. Japan 73, 1110 (1953), J.pharm. Soc. Japan 86 (1966) 544–547), dargestellt.

Nach dem Verfahren c) werden solche Verbindungen der Formel I, in denen $R^1$ Wasserstoff ist und die nach Verfahren a) oder b) hergestellt worden sind, mit einem Alkylierungsmittel Z–$R^6$, zweckmässig in Gegenwart eines Säureakzeptors und eines organischen Lösungsmittels, alkyliert.

Die Alkylierung mit einer Verbindung der Formel $Cl–(CH_2)_q–COR^4$ kann vorteilhaft für q = 2, 3 oder 4 mit einem entsprechenden Ketal der Formel

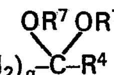

mel $Cl–(CH_2)_q–C–R^4$ ausgeführt werden, wobei $R^7$ $C_1$–$C_4$-Alkyl, $–(CH_2)_2–$, oder $–(CH_2)_3–$ ist. Anschliessend wird durch Hydrolyse mit verdünnter Säure, wie z.B. Salzsäure, Schwefelsäure oder Essigsäure, das Keton wieder freigesetzt.

Die erfindungsgemässen Verbindungen der Formel I zeigen pharmakologische Wirkungen insbesondere neuroleptische, blutdrucksenkende oder antiarrhythmische Wirkungen. So antagonisieren z.B. die Verbindungen der Beispiele 16 bis 21, 23 und 25 dosisabhängig die Amphetamin-Aggregations-Toxizität bei Mäusen. In diesem Test erhalten Gruppen von je 10 Mäusen, die auf engem Raum zusammensitzen (ca. 25 cm²/Maus), eine Stunde nach Gabe einer Verbindung der Formel I 20 mg/kg D-Amphetamin in 0,2% wässriger Lösung subcutan injiziert. Es wird dabei die Dosis der Verbindung I ermittelt, die 50% der Tiere vor dem Tod durch die Amphetaminvergiftung schützt. Die $ED_{50}$-Werte der Verbindungen der Formel I liegen zwischen 2,0 und 40 mg/kg. Weiterhin hemmen diese Verbindungen der Formel I die Bindung von Tritium-markiertem Spiroperidol an Zellmembranbestandteilen aus Homogenat von Dopamin-reichen Hirnarealen (Corpus striatum) von Ratte und Kalb ($^3H$-Spiroperiodol-Bindungstest, J.Z.Fields et al., Brain Research 136, 578 (1977) ). Die Konzentrationen der Verbindungen I, die für eine 50%ige Hemmung erforderlich sind, liegen zwischen 0,01–0,1 mMol.

Die Verbindungen haben keine oder nur eine schwache kataleptogene Wirkung, d.h. sie bewirken nur in hohen Dosen (>80 mg/kg) eine kataleptische Starre bei Ratten.

Daneben zeigen einige Verbindungen der Formel I blutdrucksenkende (Beispiel 2, 12, 13) und antiarrhythmische (Beispiel 5, 7, 11) Eigenschaften. Die blutdrucksenkenden Eigenschaften werden am normotonen narkotisierten Hund bestimmt. Der Arteriendruck wird in der A.carotis oder A.femoralis und der Venendruck in der Vena

jugularis bestimmt. Gleichzeitig werden Atemfrequenz und Atemvolumen und durch subcutan eingestochene Elektroden das EKG registriert. Ein Herzfrequenzmessgerät, das die Frequenz pro Minute angibt, wird vom EKG- oder Blutdruckmessgerät gesteuert. Wenn alle Ruhewerte bestimmt sind, wird die Substanz in einer Dosis von 1,0, 3,0 oder 5,0 mg/kg Körpergewicht intravenös appliziert und eine fortlaufende Registrierung über 30 bis 60 Minuten (bei längerer Wirksamkeit auch länger) vorgenommen.

Die antiarrhythmische Wirkung wird an normotonen, narkotisierten Hunden, die mit Strophantin vergiftet sind, gemessen. Der Blutdruck wird von der A.carotis aufgenommen, das EKG mit Einstichelektroden von den Extremitäten abgeleitet und die Herzfrequenz elektronisch aus den R-Zeichen des EKG bestimmt. Dem Hund wird dann bis zum Auftreten von ventrikulären Extrasystolen Strophantin als intravenöse Dauerinfusion über eine Beinvene zugeführt (4 mcg/kg/ml und Minute). Die Arrhythmien werden durch i.v. Injektionen einer 5% Lösung einer Verbindung der Formel I in Dosen von z.B. 1 und 3 mg/kg Körpergewicht behandelt.

Aus DE-A- 2 818 403 (A) sind 3-Piperazino-1-phenylisochinoline mit Wirkungen auf das Zentralnervensystem, z.B. mit antidepressiver Wirkung, bekannt. Diese Verbindungen unterscheiden sich von den Verbindungen der Formel I vorliegender Erfindung hauptsächlich durch den Substituenten in der 1-Stellung des Isochinolinringes. In (A) ist der Substituent ein gegebenenfalls substituierter Phenylrest, während er in den erfindungsgemässen Verbindungen entweder ein Wasserstoffatom oder einen Alkylrest bedeutet.

Die neuen Verbindungen unter der Formel I potenzieren die Wirkung von 5-Hydroxytryptophan. Die bekannten Verbindungen zeigen keine solche Aktivität.

Aus DE-A- 2 030 675 sind u.a. strukturell ähnliche 1-unsubstituierte 3-Aminoisochinoline bekannt, die jedoch in 4-Stellung phenylsubstituiert sind. Solche Verbindungen sollen u.a. «neuromuskuläre Blockierungswirkung» aufweisen und deshalb als «ZNS-Depressantien» brauchbar sein. Die Verbindungen gemäss der Erfindung zeigen eine gute, die bekannten Verbindungen jedoch keine oder nur sehr geringe neuroleptische Wirkung.

Die Verbindungen gemäss der Erfindung können entweder allein oder mit physiologisch verträglichen Hilfs- oder Trägerstoffen gemischt angewandt werden. Sie können oral, parenteral oder intravenös verabreicht werden. Für eine orale Anwendungsform werden eine Verbindung der Formel I mit pharmazeutisch üblichen Substanzen gemischt und durch übliche Methoden in geeignete Darreichungsformen gebracht, wie Tabletten, Steckkapseln, wässrige, alkoholische oder ölige Suspensionen oder wässrige, alkoholische oder ölige Lösungen. Als inerte Träger können z.B. Magnesiumcarbonat, Milchzucker oder Maisstärke unter Zusatz anderer Stoffe wie z.B. Magnesiumstearat verwendet werden. Dabei kann die Zubereitung sowohl als Trocken- oder Feuchtgranulat erfolgen. Als ölige Trägerstoffe oder Lösungsmittel kommen besonders pflanzliche Öle in Betracht wie z.B. Sonnenblumenöl oder Olivenöl.

Als Lösungsmittel der Salze mit physiologisch verträglichen Säuren der Verbindungen der Formel I für intravenöse Applikation kommen z.B. in Frage Wasser, physiologische Kochsalzlösung oder Mischungen von Wasser mit Alkoholen wie Äthanol, Propandiol oder Glycerin, daneben auch zweckmässig isotonische Zuckerlösungen wie z.B. Glukose- oder Mannitlösungen, oder auch eine Mischung aus den verschiedenen genannten Lösungsmitteln.

Die erfindungsgemässen Verbindungen und ihre pharmakologisch verträglichen Salze sind innerhalb eines breiten Dosierungsbereiches wirksam. Die Höhe der verabreichten Dosis ist abhängig von der Art der gewünschten Behandlung, von der Verabreichungsweise, vom Zustand, vom Typ und von der Grösse des zu behandelnden Säugetieres. Bei oraler Verabreichung werden befriedigende Ergebnisse mit Dosen von 0,1–100 mg einer Verbindung der Formel I pro kg Tierkörpergewicht erzielt. Beim Menschen variiert die tägliche Dosis zwischen 20 und 800 mg, vorzugsweise zwischen 50 und 500 mg, wobei Einzeldosen von 20–200 mg, vorzugsweise ein bis dreimal täglich gegeben werden können. Für intravenöse oder intramuskuläre Anwendung beträgt die Dosis 5–300 mg, vorzugsweise 10–200 mg täglich.

Insbesondere sind die folgenden Dosierungen bevorzugt: Als Antihypertonicum werden einem erwachsenen Menschen (70 kg Körpergewicht) 50–100 mg pro Tag intravenös injiziert oder 500–1000 mg pro Tag peroral appliziert.

Als Antiarrhythmikum werden einem Erwachsenen 100–500 mg pro Tag intravenös injiziert oder 500–1000 mg pro Tag peroral appliziert.

Als Neuroleptikum werden einem Erwachsenen eine Einzeldosis von 25–100 mg peroral appliziert oder 25–50 mg intravenös injiziert. Die Tagesdosis peroral beträgt 75–500 mg.

Verfahren a)

Beispiel 1:

3-(4-Methylpiperazin-1-yl)-isochinolin

6,5 g 3-Chlorisochinolin werden in 30 ml N-Methylpiperazin 48 Stunden am Rückfluss gekocht. Die Reaktionsmischung wird abgekühlt, zwischen Wasser und Toluol verteilt, die Toluolphase gründlich mit Wasser gewaschen und nach dem Trocknen das Lösungsmittel im Vakuum entfernt. Der Rückstand wird aus Diisopropyläther umkristallisiert und man erhält 3,8 g 3-(4-Methylpiperazin-1-yl)-isochinolin mit Schmp. 93–94°C, dessen Dihydrochlorid sich bei 255–257°C zersetzt.

Beispiel 2:

3-(Piperazin-1yl)-isochinolin

2,0 g 3-Chlorisochinolin werden mit 20 g Piperazin in 100 ml Diäthylenglykoldimethyläther 120 Stunden am Rückfluss gekocht. Die Aufarbeitung wie im Beispiel 1 ergibt 2,4 g 3-(Piperazin-1-yl)-isochinolin mit Schmp. 95–96°, dessen Hydrochlorid sich bei 266–268°C zersetzt.

Beispiel 3:

3- (4-(2-Hydroxyäthyl) -piperazin-1-yl) -isochinolin

In Analogie zu Beispiel 1 aus 3-Chlorisochinolin und N-(2-Hydroxyäthyl)-piperazin hergestellt. Base Schmp. 134–136°C, Dihydrochlorid Schmp. 282–283°C.

Wie vorstehend werden die 3-substituierten Isochinoline der Beispiele in Tabelle 1 aus den 3-Chlorisochinolinen und den entsprechenden Basen dargestellt.

Tabelle 1

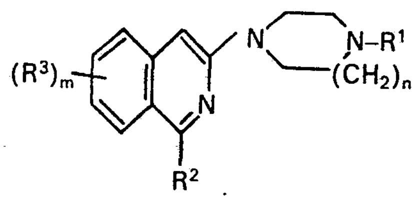

(I)

| Beispiel | m | n | $R^1$ | $R^2$ | $R^3$ | Schmp. °C/Salz (Schmp. °C) |
|---|---|---|---|---|---|---|
| 4 | 1 | 1 | 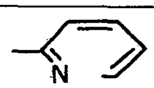 | H | H | 168–70°/2 HCl (253–256°) |
| 5 | 1 | 1 | H | H | 7–$OCH_3$ | Öl/HCl (248–251°) |
| 6 | 1 | 1 | 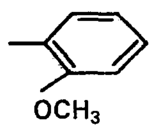 | H | H | 112–114°/HCl (135–147°) |
| 7 | 1 | 1 | –$CH_3$ | H | 6–$OCH_3$ | 102–104°/HCl (256–258°) |
| 8 | 1 | 1 | H | –$C_4H_9$ | H | Öl/HCl (160–164°) |
| 9 | 1 | 1 | H | $CH_3$ | H | Öl/HCl (252–255°, Zers.) |
| 10 | 1 | 1 | 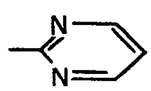 | H | H | 140–2°/2 HCl (252–255°) |
| 11 | 1 | 1 | H | H | 6–$OCH_3$ | 130–135°/HCl (220–228°, Zers.) |
| 12 | 1 | 2 | H | H | H | Öl/HCl (202–204°) |
| 13 | 1 | 1 | 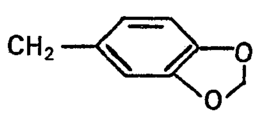 | | H | 132–134°/HCl (234–236°) |
| 14 | 1 | 1 | H | H | 7–Cl | 112–114°/HCl (318–320°) |
| 15 | 1 | 1 | H | H | 7–$OC_4H_9$ | Öl/HCl (234–235 ° |
| 16 | 1 | 1 | H | H | 7–OH | Öl/HCl (über 300 ° |
| 17 | 1 | 1 | H | H | 7–F | Öl/HCl (252–256 ° |
| 18 | 1 | 1 | H | H | 6,7-di-$OCH_3$ | Öl/HCl (220–230 ° |

Verfahren b)

Beispiel 19:

3-(4-(2-Methoxyphenyl)-piperazin-1-yl)-isochinolin

Eine Mischung aus 1,44 g 3-Aminoisochinolin, 2,5 g Bis-N,N-(2-chloroäthyl)-2-methoxyanilin, 5 g wasserfreiem Kaliumcarbonat und 30 ml Dimethylformamid wird 10 Stunden auf 130°C erhitzt. Nach dem Erkalten wird mit Wasser verdünnt, mit Methylenchlorid extrahiert und das Lösungsmittel im Vakuum entfernt. Man isoliert 1,3 g der Base mit Schmp. 112–114°C, die in seinen Eigenschaften mit der im Beispiel 6 beschriebenen Verbindung identisch ist.

Die Verbindungen der Beispiele 1–18 werden ebenfalls nach Verfahren b) mit entsprechenden Ausgangsstoffen hergestellt; die Fixpunkte sind wie vorstehend angegeben.

Beispiel 1 (b)

Aus 3-Aminoisochinolin und Bis-N,N-(2-chloroäthyl)-methylamin wird 3-(4-Methylpiperazin-1-yl)-isochinolin hergestellt.

Beispiel 2(b)

Aus 3-Aminoisochinolin und Bis-2-Chloroäthylamin wird 3-(Piperazin-1-yl)-isochinolin hergestellt.

Beispiel 3(b)

Aus 3-Aminoisochinolin und Bis-N,N-(2-chloroäthyl)-2-hydroxyäthylamin wird 3-(4-(2-Hydroxyäthyl)-piperazin-1-yl)-isochinolin hergestellt.

Beispiel 4(b)

Aus 3-Aminoisochinolin und Bis-N,N-(2-chloroäthyl)-2-pyridylamin wird 3-(4-(2-Pyridyl)-piperazin-1-yl)-isochinolin hergestellt.

Beispiel 5(b)

Aus 7-Methoxy-3-aminoisochinolin und Bis-2-chloroäthylamin wird 7-Methoxy-3-(piperazin-1-yl)-isochinolin hergestellt.

Beispiel 6(b)

Aus 3-Aminoisochinolin und Bis-N,N-(2-chloroäthyl)-2-methoxyanilin wird 3-(4-(2-Methoxyphenyl)- piperazin -1- yl)- isochinolin hergestellt. (vgl. Beispiel 19).

Beispiel 7(b)

Aus 6-Methoxy-3-aminoisochinolin und Bis-N,N-(2-chloroäthyl)-methylamin wird 6-Methoxy-3-(4-methylpiperazin-1-yl)-isochinolin hergestellt.

Beispiel 8(b)

Aus 3-Amino-1-butylisochinolin und Bis-N,N-2-chloroäthylamin wird 1-Butyl-3-(piperazin-1-yl)-isochinolin hergestellt.

Beispiel 9(b)

Aus 3-Amino-1-methylisochinolin und Bis-N,N-2-chloroäthylamin wird 1-Methyl-3-(piperazin-1-yl)-isochinolin hergestellt.

Beispiel 10(b)

Aus 3-Aminoisochinolin und Bis-N,N-(2-chloroäthyl)-2-pyrimidinylamin wird 3-(4-(2-Pyrimidinyl)-piperazin-1-yl)-isochinolin hergestellt.

Beispiel 11(b)

Aus 3-Amino-6-methoxyisochinolin und Bis-N,N-2-chloroäthylamin wird 6-Methoxy-3-(piperazin-1-yl)-isochinolin hergestellt.

Beispiel 12(b)

Aus 3-Aminoisochinolin und 2-Chloroäthyl-3-chloropropylamin wird 3-(4-Diazacycloheptan-1-yl)-isochinolin hergestellt.

Beispiel 13(b)

Aus 3-Aminoisochinolin und Bis-N,N-(2-chloroäthyl)-3,4-dioxymethylenbenzylamin wird 3- (4-(3,4- Dioxymethylenbenzyl)- piperazin-1-yl)-isochinolin hergestellt.

Beispiel 14(b)

Aus 3-Amino-7-chlor-isochinolin und Bis-N,N-2-chloroäthylamin wird 7-Chlor-3-(piperazin-1-yl)-isochinolin hergestellt.

Beispiel 15(b)

Aus 3-Amino-7-butoxy-isochinolin und Bis-N,N-2-chloroäthylamin wird 7-Butoxy-3-(piperazin-1-yl)-isochinolin hergestellt.

Beispiel 16(b)

Aus 3-Amino-7-hydroxyisochinolin und Bis-N,N-2-chloräthylamin wird 7-Hydroxy-3-(piperazin-1-yl)-isochinolin hergestellt.

Beispiel 17(b)

Aus 3-Amino-7-fluorisochinolin und Bis-N,N-2-chloroäthylamin wird 7-Fluor-3-(piperazin-1-yl)-isochinolin hergestellt.

Beispiel 18(b)

Aus 3-Amino-6,7-dimethoxy-isochinolin und Bis-N,N-2-chloroäthylamin wird 6,7-Dimethoxy-3-(piperazin-1-yl)-isochinolin hergestellt.

Verfahren c)

Beispiel 20

3-(4-(3-(4-Fluorbenzoyl)- propyl)-piperazin-1-yl)-isochinolin

6,4 g 3-(Piperazin-1-yl)-isochinolin, hergestellt nach Beispiel 2 (Verfahren a oder 2 (b) Verfahren b), 13,5 g ω-Chlor-4-fluorbutyrophenon-äthylenketal, 10,6 g Natriumcarbonat und 1 g Natriumjodid werden in 100 ml Dimethylformamid auf 90–100°C für 4 Stunden erhitzt. Das Reaktionsgemisch wird abgekühlt und zwischen Chloroform und Wasser verteilt. Die Chloroformphase wird mehrmals mit Wasser extrahiert und dann im Vakuum das Lösungsmittel abgedampft.

Der Rückstand wird in 100 ml Äthanol gelöst und mit 150 ml halkonzentrierter Salzsäure 3 Stunden gekocht. Nach dem Abziehen des Alkohols im Vakuum wird die Lösung mit verdünnter Natronlauge alkalisch gestellt und mit Chloroform extrahiert.

Aus den vereinigten Chloroformextrakten werden 4,6 g des 3-(4-(3-(4-Fluorbenzoyl)-propyl)-piperazin-1-yl)-isochinolin mit Schmp. 148–150°C isoliert. Hydrochlorid Schmp. 215–218°C.

Beispiel 21

Aus 3-(Piperazin-1-yl)-isochinolin und ω-Chlor - 2,4 - difluorbutyrophenon - äthylenketal wird 3-(4-[3-(2,4-Difluorbenzoyl)-propyl]-piperazin-1-yl)-isochinolin hergestellt, Schmp. 122–124°C, Hydrochlorid Schmp. 240–242°C.

Beispiel 22

Au 7-Methoxy-3 -(piperazin-1-yl)- isochinolin und ω-Chlor-4-fluorbutyrophenon-äthylenketal wird 7-Methoxy-3-(4-[3-(4-fluorbenzoyl)-propyl]-piperazin-1-yl)-isochinolin hergestellt, Schmp. 182–185°C, Hydrochlorid Schmp. 198–200°C.

Beispiel 23

Aus 7-Chlor-3 -(piperazin-1-yl)- isochinolin und ω-Chlor-4-fluorbutyrophenon-äthylenketal wird 7-Chlor- 3-(4-(3-(4-Fluorbenzoyl) -propyl)-piperazin-1-yl)-isochinolin hergestellt, Öl, Hydrochlorid Schmp. 198–202°C.

Beispiel 24

Aus 2-Methyl-3-(piperazin-1-yl)-isochinolin und ω-Chlor-4-fluorbutyrophenon-ethylenketyl wird 1-Methyl-3 -(4-[3-(4-fluorbenzoyl)- propyl]-piperazin-1-yl)-isochinolin hergestellt, Schmp. 112–115°C, Hydrochlorid Schmp. 250–254°C.

Beispiel 25

Aus 3-(4-Diazacycloheptan-1-yl)-isochinolin und ω-Chlor-4-fluorbutyrophenon-äthylenketal wird 3-(4-(3-(4-Fluorbenzoyl)-propyl)-diazacycloheptan-1-yl)-isochinolin hergestellt, Öl, Hydrochlorid Schmp. 206–208°C.

Beispiel 26

Aus 3-(Piperazin-1-yl)-isochinolin und ω-Chlor-2-nitro-4-fluorbutyrophenon-äthylenketal wird 3-(4-(3-(2-Nitro-4-fluorbenzoyl)-propyl)-piperazin-1-yl)-isochinolin hergestellt, Schmp. 103–105°C.

Beispiel 27

3-(4-(3-(2-Amino-4- fluorbenzoyl)- propyl)-piperazin-1-yl)-isochinolin

1,7 g 3-(3-(4-(2-Nitro-4-fluorbenzoyl)-propyl)-piperazin-1-yl)-isochinolin (Beispiel 26) werden mit 180 mg 10%igem Palladium auf Kohle in 8 ml äthanolischer Chlorwasserstofflösung und 50 ml Methanol bei Normaldruck und Raumtemperatur hydriert bis die theoretische Menge Wasserstoff aufgenommen ist. Der Katalysator wird abfiltriert, das Lösungsmittel verdampft und der Rückstand aus Äthanol umkristallisiert.

Schmp. 124–128°C, Hydrochlorid Schmp. 220–223°C.

Beispiel 28

3-(4-(3-(5-Fluorbenzisoxazol -3-yl)-propyl)-piperazin-1-yl)-isochinolin

a) 1,2 g 3-(4-(3-(2,4-Difluorbenzoyl)-propyl)-piperazin-1-yl)-isochinolin, 1,2 g Ammoniumacetat, 0,6 g Hydroxylaminhydrochlorid werden in 15 ml Methanol und 4 ml Wasser 2 1/2 Stunden am Rückfluss gekocht. Nach dem Abkühlen wird mit Wasser verdünnt und mit Chloroform extrahiert. Man isoliert 0,85 g Oxim mit Schmp. 144–146°C.

b) Zu 0,25 g Natriumhydrid (50%) in 20 ml Tetrahydrofuran werden bei Raumtemperatur langsam 0,8 g des oben dargestellten Oxims in 15 ml DMF und 8 ml THF zugetropft. Anschliessend wird die Suspension nach 6 Stunden bei 80–90°C gerührt und dann mit Wasser hydrolysiert. Die Lösung wird zwischen gesättigter Kochsalzlösung und Äther verteilt. Aus der Ätherlösung kristallisieren 0,1 g mit Schmp. 130–133°C. Hydrochlorid Schmp. 172–174°C.

Beispiel 29

3-(4-(4-Methoxybenzoyl)-piperazin-1-yl)-isochinolin

10 g 3-(Piperazin-1-yl)-isochinolin in 140 ml Pyridin gelöst werden tropfenweise mit 9,38 g 4-Methoxybenzoylchlorid versetzt. Nach 1 Stunde Rühren bei Raumtemperatur wird mit Wasser verdünnt und mit Essigester extrahiert. Die Essigester-Phase wird abgetrocknet und das Lösungsmittel abgedampft. Es können 5,8 g mit Schmp. 156–159°C isoliert werden.

Analog werden folgende Verbindungen hergestellt:

Beispiel 30

Aus 7-Butoxy-3-(piperazin-1-yl)-isochinolin und ω-Chlor-4-fluorbutyrophenon-äthylenketal wird 7-Butoxy-3-(4-(3-(4-fluorbenzoyl)-propyl)- piperazin-1-yl)- isochinolin hergestellt. Schmp. 157–159°C.

Beispiel 31

Aus 7-Fluor-3-(piperazin-1-yl)-isochinolin und ω-Chlor-4-fluorbutyrophenon-äthylenketal wird 7-Fluor-3-(4-(3-(4-fluorbenzoyl)-propyl)-piperazin-1-yl)-isochinolin hergestellt. Hydrochlorid Schmp. 212–217°C.

Beispiel 32

Aus 3-(Piperazin-1-yl)-isochinolin und ω-Chlor-2-thienylpropylketan-äthylenketal wird 3-(4-(3-(2-Thienoyl)- propyl)- piperazin-1- yl)-isochinolin hergestellt. Schmp. 94–100°C. Hydrochlorid Schmp. 254–260°C.

Beispiel 33

Aus 3-(Piperazin-1-yl)-isochinolin und ω-Chlor-4-brombutyrophenon-äthylenketal wird 3-(4-(3-(4-Brombenzoyl)- propyl)- piperazin-1-yl)-isochinolin hergestellt. Öl, Hydrochlorid Schmp. 250–254°C.

Beispiel 34

Aus 3-(Piperazin-1-yl)-isochinolin und ω-Chlor-3-benzoylpropyl-äthylenketal wird 3-(4-(3-Benzoylpropyl)- piperazin-1- yl)- isochinolin hergestellt, Öl, Hydrochlorid Schmp. 277–278°C.

**Patentansprüche für die Vertragsstaaten: BE-CH-DE-FR-GB-IT-Li-LU-NL-SE**

1. Isochinolinderivate der allgemeinen Formel I

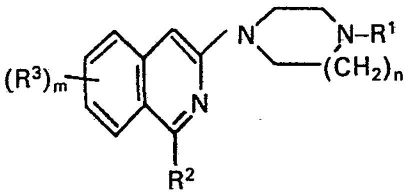

(I)

worin bedeuten:

m eins oder zwei, n eins oder zwei,

$R^1$ a) Wasserstoff,

b) $C_1$–$C_6$-Alkyl, geradkettig oder verzeigt, gegebenenfalls substituiert mit Hydroxy, $C_1$–$C_4$Alkoxy- oder $C_3$–$C_6$-Cycloalkyl,

c) Thienyl, Furyl, Pyridyl, Pyrimidyl,

d) Formyl oder $C_1$—$C_6$-Alkoxycarbonyl,

e) Phenyl, das einfach, zweifach oder dreifach substituiert sein kann mit $C_1$—$C_6$-Alkyl, $C_1$–$C_6$-Alkoxy, Methylendioxy, Hydroxy, Nitro, Amino, Trifluormethyl oder Halogen,

f) $-(CH_2)_q-COR^4$, worin q 0, 1, 2, 3 oder 4 und $R^4$ Thienyl, Furyl, Pyridyl oder ein wie unter e) angegebener, gegebenenfalls substituierter Phenylrest ist,

g) 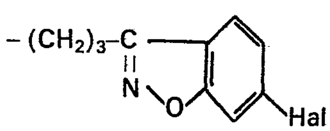 ,

wobei Hal Halogen bedeutet;

$R^2$ Wasserstoff oder $C_1$–$C_6$-Alkyl,

$R^3$ Wasserstoff, Halogen, Hydroxy-, $C_1$–$C_6$-Alkyl, $C_1$–$C_6$-Alkoxy, Benzyloxy, Methylendioxy, Äthylendioxy, Nitro oder Amino, sowie deren Salze mit physiologisch verträglichen Säuren.

2. Verfahren zur Herstellung eines Isochinolinderivates der Formel I in Anspruch 1, dadurch gekennzeichnet, dass man a) eine Verbindung der Formel II

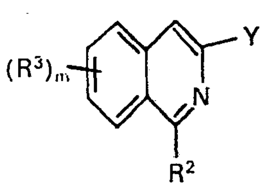

(II)

worin Y Halogen, Alkyloxy oder Alkylthio mit jeweils 1–4 C-Atomen im Alkylrest, ist und $R^2$, $R^3$ und m die zur Formel I in Anspruch 1 genannten Bedeutungen haben, mit einem Amin der Formel III

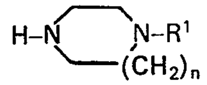

(III)

worin $R^1$ und n die zur Formel I genannten Bedeutungen haben, umsetzt,

b) eine Verbindung der Formel IV

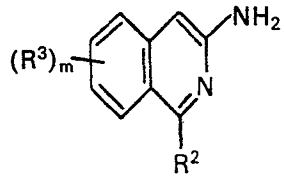

(IV)

worin $R^2$, $R^3$ und m die zur Formel I in Anspruch 1 genannten Bedeutungen haben, mit einer Verbindung der Formel V

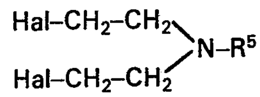

(V)

worin Hal Halogen, und $R^5$ Wasserstoff, $C_1$–$C_6$-Alkyl oder einen wie in Formel I unter $R^1$c) oder $R^1$e) angegebenen Rest bedeutet, oder

c) eine Verbindung der Formel Ia

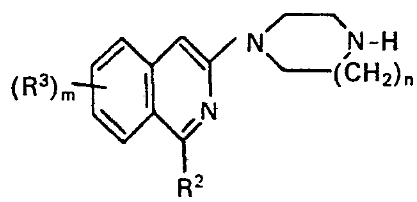

(Ia)

worin $R^2$, $R^3$, m und n die zur Formel I in Anspruch 1 genannten Bedeutungen haben, mit einem Alkylierungsmittel der Formel $Z-R^6$, worin Z Jod, Chlor oder Brom und $R^6$ einen wie in Formel I unter $R^1$b angegebenen Alkylrest bedeutet, oder mit einem Chlorameisensäureester der Formel $Cl-CO_2-(C_1-C_6)$-Alkyl oder mit einer Verbindung der Formel $Cl-(CH_2)_q-COR^4$, worin $R^4$ und q die zur Formel I unter $R^1$f) genannten Bedeutungen haben, umsetzt.

3. Isochinolinderivate der Formel I zur Anwendung in einem therapeutischen Verfahren.

4. Pharmazeutische Zubereitung, dadurch gekennzeichnet, dass sie eine Verbindung der Formel I enthält oder aus dieser besteht.

5. Verfahren zur Herstellung einer pharmazeutischen Zubereitung nach Anspruch 4, dadurch gekennzeichnet, dass man eine Verbindung der Formel I, gegebenenfalls zusammen mit einem üblichen pharmazeutischen Trägerstoff und/oder Hilfsstoff in eine für pharmazeutische Zwecke geeignete Darreichungsform bringt.

6. Verbindung der Formel I in Anspruch 1 zur Verwendung als Neuroleptikum, Antihypertonikum oder Antiarhythmikum.

**Patentanspruch für den Vertragsstaat: AT**

1. Verfahren zur Herstellung von Isochinolinderivaten der allgemeinen Formel I

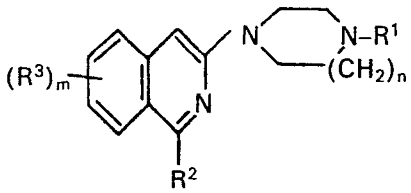

(I)

worin bedeuten:

m eins oder zwei, n eins oder zwei,

$R^1$ a) Wasserstoff,

b) $C_1$–$C_6$-Alkyl, geradkettig oder verzweigt, gegebenenfalls substituiert mit Hydroxy, $C_1$–$C_4$-Alkoxy- oder $C_3$–$C_6$-Cycloalkyl,

c) Thienyl, Furyl, Pyridyl, Pyrimidyl,

d) Formyl oder $C_1$–$C_6$-Alkoxycarbonyl,

e) Phenyl, das einfach, zweifach oder dreifach substituiert sein kann mit $C_1$–$C_6$-Alkyl, $C_1$–$C_6$-Alkoxy, Methylendioxy, Hydroxy, Nitro, Amino, Trifluormethyl oder Halogen,

f) $-(CH_2)_q-COR^4$, worin q 0, 1, 2, 3 oder 4 und $R^4$ Thienyl, Furyl, Pyridyl oder ein wie unter e) angegebener, gegebenenfalls substituierter Phenylrest ist,

g) 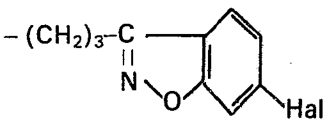 ,

wobei Hal Halogen bedeutet;

$R^2$ Wasserstoff oder $C_1$–$C_6$-Alkyl,

$R^3$ Wasserstoff, Halogen, Hydroxy-, $C_1$–$C_6$-Alkyl, $C_1$–$C_6$-Alkoxy, Benzyloxy, Methylendioxy, Äthylendioxy, Nitro oder Amino, sowie von deren Salzen mit physiologisch verträglichen Säuren, dadurch gekennzeichnet, dass man

a) eine Verbindung der Formel II

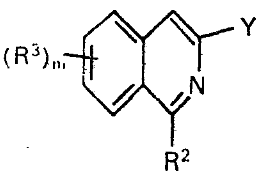

(II)

worin Y Halogen, Alkyloxy oder Alkylthio mit jeweils 1–4 C-Atomen im Alkylrest, ist und $R^2$, $R^3$ und m die zur Formel I in Anspruch 1 genannten Bedeutungen haben, mit einem Amin der Formel III

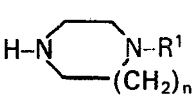

(III)

worin $R^1$ und n die zur Formel I genannten Bedeutungen haben, umsetzt,

b) eine Verbindung der Formel IV

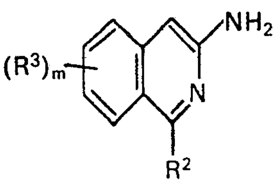

(IV)

worin $R^2$, $R^3$ und m die zur Formel I in Anspruch 1 genannten Bedeutungen haben, mit einer Verbindung der Formel V

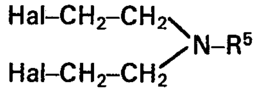

(V)

worin Hal Halogen, und $R^5$ Wasserstoff, $C_1$–$C_6$-Alkyl oder einen wie in Formel I unter $R^1$c) oder $R^1$e) angegebenen Rest bedeutet, oder

c) eine Verbindung der Formel Ia

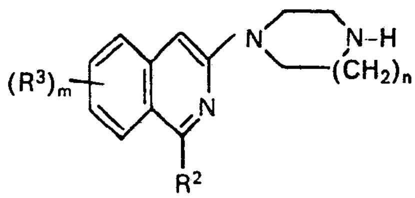

(Ia)

worin $R^2$, $R^3$, m und n die zur Formel I in Anspruch 1 genannten Bedeutungen haben, mit einem Alkylierungsmittel der Formel $Z-R^6$, worin Z Jod, Chlor oder Brom und $R^6$ einen wie in Formel I unter $R^1$b angegebenen Alkylrest bedeutet, oder mit einem Chlorameisensäureester der Formel $Cl-CO_2-(C_1-C_6)$-Alkyl oder mit einer Verbindung der Formel $Cl-(CH_2)_q-COR^4$, worin $R^4$ und q die zur Formel I unter $R^1$f) genannten Bedeutungen haben, umsetzt und gegebenenfalls durch Zusatz einer physiologisch verträglichen Säure ein Salz herstellt.

**Revendications pour les Etats contractants: BE-CH-DE-FR-GB-IT-LI-LU-NL-SE**

1. Dérivés de l'isoquinoléine répondant à la formule générale

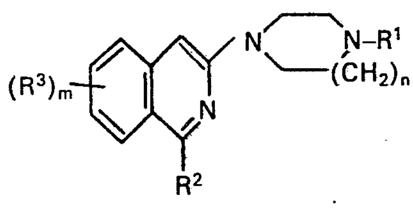

(I)

dans laquelle

m est égal à 1 ou 2, n à 1 ou 2,

$R^1$ est a) l'hydrogène,

b) un alcoyle en $C_1$ à $C_6$, linéaire ou ramifié éventuellement substitué par un groupe hydroxy, alcoxy en $C_1$ à $C_4$ ou cycloalcoyle en $C_3$ à $C_6$,

c) un groupe thiényle, furyle, pyridyle, pyrimidyle,

d) un groupe formyle ou (alcoxy en $C_1$–$C_6$)carbonyle,

e) un groupe phényle, qui peut être mono-, di- ou trisubstitué avec un groupe alcoyle en $C_1$ à $C_6$, alcoxy en $C_1$ à $C_6$, méthylènedioxy, hydroxy, nitro, amino, trifluorométhyle ou un halogène,

f) $-(CH_2)_q-COR^4$, où q est égal à 0, 1, 2, 3 ou 4 et $R_4$ est un groupe thiényle, furyle, pyridyle ou un radical phényle tel qu'indiqué en e), éventuellement substitué,

g) 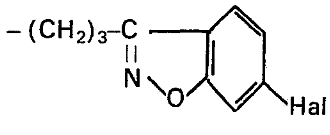 ,

où Hal désigne un halogène;

$R^2$ est l'hydrogène ou un groupe alcoyle en $C_1$ à $C_6$,

$R^3$ est l'hydrogène, un halogène, un groupe hydroxy, alcoyle en $C_1$ à $C_6$, alcoxy en $C_1$ à $C_6$, benzyloxy, méthylènedioxy, éthylènedioxy, nitro ou amino, ainsi que leurs sels avec des acides physiologiquement acceptables.

2. Procédé de préparation de dérivés d'isoquinoléine selon la revendication 1, caractérisé en ce qu'on fait réagir:

a) un composé répondant à la formule II

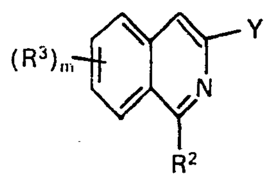

(II)

dans laquelle Y est un halogène, un groupe alcoyloxy ou alcoylthio avec à chaque fois 1 à 4 atomes de carbone dans le radical alcoyle et $R^2$, $R^3$ et m ont les significations indiquées pour la formule I dans la revendication 1, avec une amine répondant à la formule III

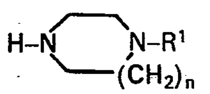

(III)

dans laquelle $R^1$ et n ont les significations indiquées pour la formule I,

b) on fait réagir un composé répondant à la formule IV

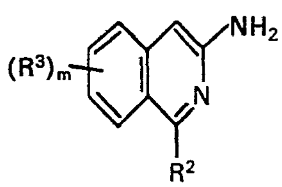

(IV)

dans laquelle $R^2$, $R^3$ et m ont les signification indiquées pour la formule I dans la revendication 1 avec un composé répondant à la formule V

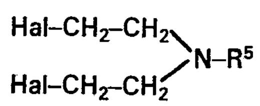

(V)

dans laquelle Hal est un halogène et $R^5$ l'hydrogène, un groupe alcoyle en $C_1$ à $C_6$ ou un radical tel qu'indiqué pour la formule I en $R^1$c) ou $R^1$e), ou

c) on fait réagir un composé répondant à la formule

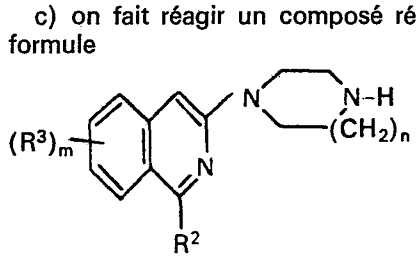

(Ia)

dans laquelle $R^2$, $R^3$, m et n ont les significations indiquées pour la formule I dans la revendication 1 avec un agent d'alcoylation répondant à la formule $Z-R^6$, dans laquelle Z désigne l'iode, le chlore ou le brome et $R^6$ un radical alcoyle tel qu'indiqué pour la formule I en $R^1$b), ou avec un ester chloroformique répondant à la formule $Cl-CO_2$-(alcoyle en $C_1$ à $C_6$) ou avec un composé répondant à la formule $Cl-(CH_2)_q-COR^4$, dans laquelle $R^4$ et q ont les significations indiquées pour la formule I en $R^1$f), et le cas échéant on prépare un sel par addition d'un acide physiologiquement acceptable.

3. Dérivés d'isoquinoléine de formule I destinés à être utilisés dans une méthode thérapeutique.

4. Composition pharmaceutique caractérisée en ce qu'elle contient ou se compose d'un composé de formule I.

5. Procédé de préparation d'une composition pharmaceutique selon la revendication 4, caractérisé en ce que l'on met un composé de formule I, éventuellement en mélange avec un véhicule et/ou auxiliaire pharmaceutique usuel sous une forme d'administration convenant à un but pharmaceutique.

6. Composés de formule I selon la revendication 1 destinés à être utilisés comme neuroleptiques antihypertoniques ou antiarythmiques.

**Revendication pour l'Etat contractant: AT**

Procédé de préparation de dérivés de l'isoquinoléine répondant à la formule générale I

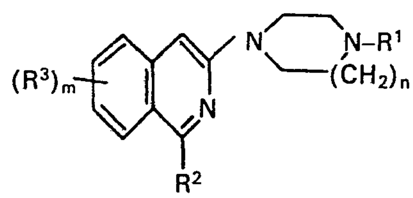

(I)

dans laquelle

m est égal à 1 ou 2, n à 1 ou 2,

$R^1$ est a) l'hydrogène,

b) un alcoyle en $C_1$ à $C_6$, linéaire ou ramifié éventuellement substitué par un groupe hydroxy, alcoxy en $C_1$ à $C_4$ ou cycloalcoyle en $C_3$ à $C_6$,

c) un groupe thiényle, furyle, pyridyle, pyrimidyle,

d) un groupe formyle ou (alcoxy en $C_1$–$C_6$) carbonyle,

e) un groupe phényle, qui peut être mono-, di- ou trisubstitué avec un groupe alcoyle en $C_1$ à $C_6$, alcoxy en $C_1$ à $C_6$, méthylènedioxy, hydroxy, nitro, amino, trifluoro-méthyle ou un halogène,

f) $-(CH_2)_q-COR^4$, où q est égal à 0, 1, 2, 3 ou 4 et $R_4$ est un groupe thiényle, furyle, pyridyle ou un radical phényle tel qu'indiqué en e), éventuellement substitué,

g) 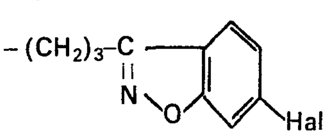 ,

où Hal désigne un halogène;

$R^2$ est l'hydrogène ou un groupe alcoyle en $C_1$ à $C_6$,

$R^3$ est l'hydrogène, un halogène, un groupe

hydroxy, alcoyle en $C_1$ à $C_6$, alcoxy en $C_1$ à $C_6$, benzyloxy, méthylènedioxy, éthylènedioxy, nitro ou amino, ainsi que leurs sels avec des acides physiologiquement acceptables.

2. Procédé de préparation de dérivés d'isoquinoléine selon la revendication 1, caractérisé en ce qu'on fait réagir:

a) un composé répondant à la formule II

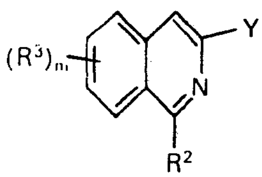
(II)

dans laquelle Y est un halogène, un groupe alcoyloxy ou alcoylthio avec à chaque fois 1 à 4 atomes de carbone dans le radical alcoyle et $R^2$, $R^3$ et m ont les significations indiquées pour la formule I dans la revendication 1, avec une amine répondant à la formule III

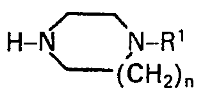
(III)

dans laquelle $R^1$ et n ont les significations indiquées pour la formule I,

b) on fait réagir un composé répondant à la formule IV

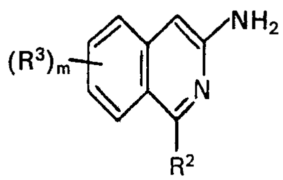
(IV)

dans laquelle $R^2$, $R^3$ et m ont les significations indiquées pour la formule I dans la revendication 1 avec un composé répondant à la formule V

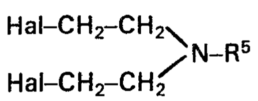
(V)

dans laquelle Hal est un halogène et $R^5$ l'hydrogène, un groupe alcoyle en $C_1$ à $C_6$ ou un radical tel qu'indiqué pour la formule I en $R^1$c) ou $R^1$e), ou

c) on fait réagir un composé répondant à la formule

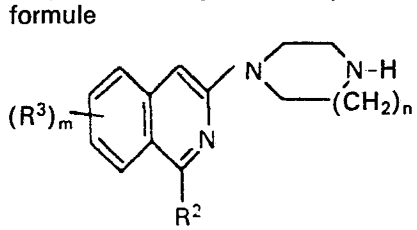
(Ia)

dans laquelle $R^2$, $R^3$, m et n ont les significations indiquées pour la formule I dans la revendication 1 avec un agent d'alcoylation répondant à la formule $Z-R^6$, dans laquelle Z désigne l'iode, le chlore ou le brome et $R^6$ un radical alcoyle tel qu'indiqué pour la formule I en $R^1$b), ou avec un ester chloroformique répondant à la formule Cl–$CO_2$-(alcoyle en $C_1$ à $C_6$) ou avec un composé répondant à la formule $Cl-(CH_2)_q-COR^4$, dans laquelle $R^4$ et q ont les significations indiquées pour la formule I en $R^1$f), et le cas échéant on prépare un sel par addition d'un acide physiologiquement acceptable.

**Claims for the Contracting States: BE, CH, DE, FR, GB, IT, LI, LU, NL, SE**

1. An isoquinoline derivative of the general formula I

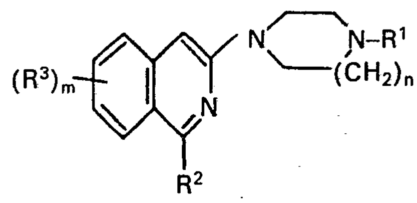
(I)

in which m denotes one or two, n denotes one or two, $R^1$ denotes a) hydrogen, b) straight-chain or branched $C_1$–$C_6$-alkyl, optionally substituted by hydroxyl, $C_1$–$C_4$-alkoxy or $C_3$–$C_6$-cycloalkyl, c) thienyl, furyl, pyridyl or pyrimidyl, d) formyl or $C_1$–$C_6$-alkoxycarbonyl, e) phenyl, which can be monosubstituted, disubstituted or trisubstituted by $C_1$–$C_6$-alkyl, $C_1$–$C_6$-alkoxy, methylenedioxy, hydroxyl, nitro, amino, trifluoromethyl or halogen, f) $-(CH_2)_q-COR^4$, in which q is 0, 1, 2, 3 or 4 and $R^4$ is thienyl, furyl, pyridyl or an optionally substituted phenyl radical as indicated under e) and g)

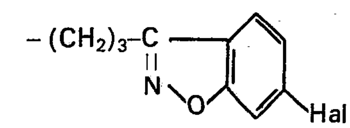
,

in which Hal denotes halogen; $R^2$ denotes hydrogen or $C_1$–$C_6$-alkyl, and $R^3$ denotes hydrogen, halogen, hydroxyl, $C_1$–$C_6$-alkyl, $C_1$–$C_6$-alkoxy, benzyloxy, methylenedioxy, ethylenedioxy, nitro or amino, and a salt thereof with a physiologically acceptable acid.

2. A process for the preparation of an isoquinoline derivative of the formula I in claim 1, which comprises a) reacting a compound of the formula II

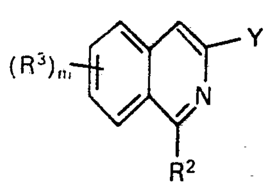
(II)

in which Y is halogen or alkyloxy or alkylthio, each of which has 1–4 C atoms in the alkyl radical, and $R^2$, $R^3$ and m have the meanings mentioned for the formula I in chlaim 1, with an amine of the formula III

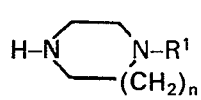
(III)

in which $R^1$ and n have the meanings mentioned for the formula I,

b) reacting a compound of the formula IV

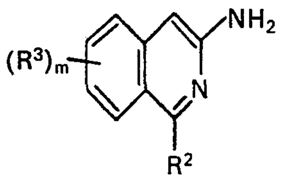

(IV)

in which $R^2$, $R^3$ and m have the meanings mentioned for the formula I in claim 1, with a compound of the formula V

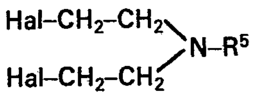

(V)

in which Hal denotes halogen and $R^5$ denotes hydrogen, $C_1$–$C_6$-alkyl or a radical as defined in formula I under $R^1$c) or $R^1$e), or

c) reacting a compound of the formula Ia

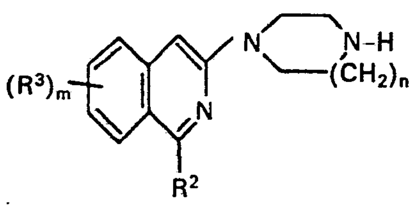

(Ia)

in which $R^2$, $R^3$, m and n have the meanings mentioned for the formula I in claim 1, with an alkylating agent of the formula Z–$R^6$ in which Z denotes iodine, chlorine or bromine and $R^6$ denotes an alkyl radical as indicated in formula I under $R^1$b, or with a chloroformic acid ester of the formula Cl–$CO_2$–($C_1$–$C_6$)-alkyl or with a compound of the formula Cl–$(CH_2)_q$–$COR^4$ in which $R^4$ and q have the meanings mentioned for the formula I under $R^1$f).

3. Isoquinoline derivatives of formula I for the use in a therapeutical treatment.

4. Pharmaceutical composition containing or consisting of a compound of the formula I.

5. Process for the manufacture of a pharmaceutical composition according to claim 4, which comprises bringing into a form suitable for pharmaceutical purposes a compound of formula I, if necessary together with a suitable pharmaceutical carrier and/or auxiliary.

6. A compound of formula I as given in claim 1 for application as a neuroleptic, antihypertonic or antiarrhythmic remedy.

**Claim for the Contracting State: AT**

A process for the manufacture of isoquinoline derivatives of the feneral formula I

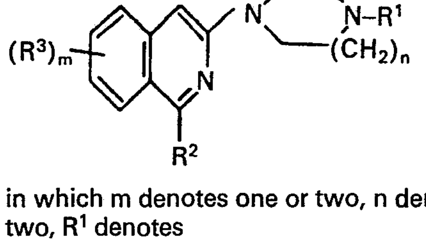

(I)

in which m denotes one or two, n denotes one or two, $R^1$ denotes

a) hydrogen,

b) straight-chain or branched $C_1$–$C_6$-alkyl, optionally substituted by hydroxyl, $C_1$–$C_4$-alkoxy or $C_3$–$C_6$-cycloalkyl,

c) thienyl, furyl, pyridyl or pyrimidyl,

d) formyl or $C_1$–$C_6$-alkoxycarbonyl,

e) phenyl, which can be monosubstituted, disubstituted or trisubstituted by $C_1$–$C_6$-alkyl, $C_1$–$C_6$-alkoxy, methylenedioxy, hydroxyl, nitro, amino, trifluoromethyl or halogen,

f) –$(CH_2)_q$–$COR^4$, in which q is 0, 1, 2, 3 or 4 and $R^4$ is thienyl, furyl, pyridyl or an optionally substituted phenyl radical as indicated under e), and

g) 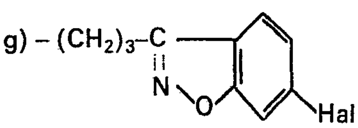 ,

in which Hal denotes halogen; $R^2$ denotes hydrogen or $C_1$–$C_6$-alkyl, and $R^3$ denotes hydrogen, halogen, hydroxyl, $C_1$–$C_6$-alkyl, $C_1$–$C_6$-alkoxy, benzyloxy, methylenedioxy, ethylenedioxy, nitro or amino, and a salt thereof with a physiologically acceptable acid, which comprises a) reacting a compound of the formula II

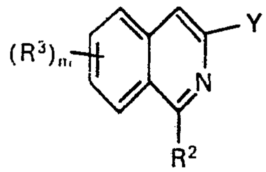

(II)

in which Y is halogen or alkyloxy or alkylthio, each of which has 1–4 C atoms in the alkyl radical, and $R^2$, $R^3$ and m have the meanings mentioned for the formula I in claim 1, with an amine of the formula III

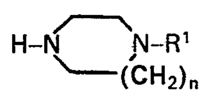

(III)

in which $R^1$ and n have the meanings mentioned for the formula I,

b) reacting a compound of the formula IV

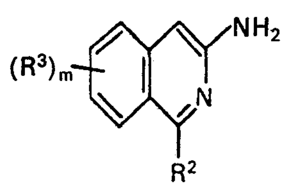

(IV)

in which $R^2$, $R^3$ and m have the meanings mentioned for the formula I in claim 1, with a compound of the formula V

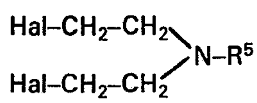

(V)

in which Hal denotes halogen and $R^5$ denotes hydrogen, $C_1$–$C_6$-alkyl or a radical as defined in formula I under $R^1$c) or $R^1$e), or

c) reacting a compound of the formula Ia

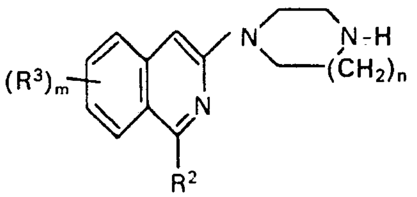

(Ia)

in which $R^2$, $R^3$, m and n have the meanings mentioned for the formula I in claim 1, with an alkylating agent of the formula $Z–R^6$ in which Z denotes iodine, chlorine or bromine and $R^6$ denotes an alkyl radical as indicated in formula I under $R^1$b), or with a chloroformic acid ester of the formula $Cl–CO_2–(C_1–C_6)$-alkyl or with a compound of the formula $Cl–(CH_2)_q–COR^4$ in which $R^4$ and q have the meanings mentioned for the formula I under $R^1$f), and, if desired, reacting the compound thus obtained with a physiologically acceptable acid.